# EUROPEAN PATENT APPLICATION

(11) **EP 2 293 098 A1**
(43) Date of publication of application: **09.03.2011**
(21) Application number: 10174357.3
(22) Date of filing: 27.08.2010
(51) Int. Cl.: G01S 7/52, G01S 15/89, G10K 11/34, G06T 7/00

(54) **Steering angle adjustment of scan lines using virtual transducer elements in an ultrasound system**

(30) Priority: 31.08.2009 KR 20090081265
(71) Applicant: Medison Co., Ltd., Kangwon-do 250-870 (KR)
(72) Inventor: Shin, Dong Kuk, 135-851 Seoul (KR); Kim, Jong Sik, 135-851 Seoul (KR)
(74) Representative: Schmid, Wolfgang

(57) **Abstract**

Embodiments forming an ultrasound image by adjusting a steering angle (θ) of scan lines using virtual transducer elements (132B) in an ultrasound system are disclosed herein. In one embodiment, a processing unit forms a first ultrasound image by using the ultrasound data, which may be acquired based on first scan lines steered at a first steering angle. The processing unit determines a center of the target object (VC) on the first ultrasound image. A control unit defines virtual transducer elements (132B) associated with an array transducer (132A), defines second scan lines and computes a second steering angle (θ) of the second scan lines based on the virtual transducer elements (132B) and the center of the target object (VC). An ultrasound data acquisition unit forms second ultrasound data based on the second scan lines steered at the second steering angle (θ). The processing unit forms a second ultrasound image by using the second ultrasound data.

## Description

### TECHNICAL FIELD

Embodiments described herein generally relate to an ultrasound system, and more particularly to steering angle adjustment of scan lines using virtual transducer elements in an ultrasound system.

### BACKGROUND

An ultrasound system has been extensively used in the medical field due to its non-invasive and non-destructive nature. Modem high-performance ultrasound imaging diagnostic systems and techniques are commonly used to produce two-dimensional or three-dimensional ultrasound images of internal features of patients.

The ultrasound system employs an ultrasound probe containing a transducer array for transmission and reception of ultrasound signals. The ultrasound signals are transmitted along scan lines aligned with the direction of a scan head of the ultrasound probe. The ultrasound system forms ultrasound images based on the received ultrasound signals. Recently, the technique of transmitting the ultrasound signals by steering the scan lines has been in use to obtain an ultrasound image having a wider view angle. In this case, however, since the length of the transducer array is fixed, the maximum steering angle may be limited.

Embodiments for forming an ultrasound image by adjusting a steering angle of scan lines using virtual transducer elements in an ultrasound system are disclosed herein. In one embodiment, by way of non-limiting example, an ultrasound system comprises: an ultrasound data acquisition unit configured to transmit ultrasound signals to a target object along first scan lines steered at a first steering angle and receive echo signals reflected from the target object to form first ultrasound data, the ultrasound data acquisition unit including an array transducer containing a plurality transducer elements; a processing unit configured to form a first ultrasound image by using the first ultrasound data and determine a center of the target object on the first ultrasound image; and a control unit configured to define virtual transducer elements associated with the array transducer, define second scan lines and compute a second steering angle of second scan lines based on the virtual transducer elements and the center of the target object, wherein the ultrasound data acquisition unit is further configured to transmit ultrasound signals to the target object along the second scan lines steered at the second steering angle and receive echo signals reflected from the target object to form second ultrasound data, and wherein the processing unit is further configured to form a second ultrasound image by using the second ultrasound data.

In another embodiment, there is provided a method of forming an ultrasound image by adjusting a steering angle of scan lines using virtual transducer elements in an ultrasound system having an array transducer containing a plurality of transducer elements, comprising: a) transmitting ultrasound signals to a target object along first scan lines originating from a first aperture including predetermined transducer elements and steered at a first steering angle, and receiving echo signals reflected from the target object to form first ultrasound data; b) forming a first ultrasound image by using the first ultrasound data and determining a center of the target object on the first ultrasound image; c) defining virtual transducer elements associated with the array transducer, defining second scan lines and computing a second steering angle of the second scan lines based on the virtual transducer elements and the center of the target object; d) transmitting ultrasound signals along the second scan lines originating from a second aperture including the predetermined transducer elements and the virtual transducer elements and steered at the second steering angle into the target object, and receiving echo signals reflected from the target object to form second ultrasound data; and e) forming a second ultrasound image by using the second ultrasound data.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

- FIG. 1: is a block diagram showing an illustrative embodiment of an ultrasound system.
- FIG. 2.: is a block diagram showing an illustrative embodiment of an ultrasound data acquisition unit.
- FIG. 3: is a block diagram showing an illustrative embodiment of a process of forming an ultrasound image by adjusting a steering angle using virtual transducer elements.
- FIG. 4: is a schematic diagram showing scan lines extending from an aperture of an array transducer and steered at a predetermined steering angle.
- FIG. 5: is a schematic diagram showing an embodiment of determining a center of a target object by defining a region of interest on an ultrasound image.
- FIG. 6: is a schematic diagram showing an embodiment of determining a center of a target object by using a seed point on an ultrasound image.
- FIG. 7: is a schematic diagram showing an embodiment of determining a steering angle of scan lines by using virtual transducer elements.
- FIG. 8: is a schematic diagram showing an embodiment of performing receive focusing upon receive signals.

### DETAILED DESCRIPTION

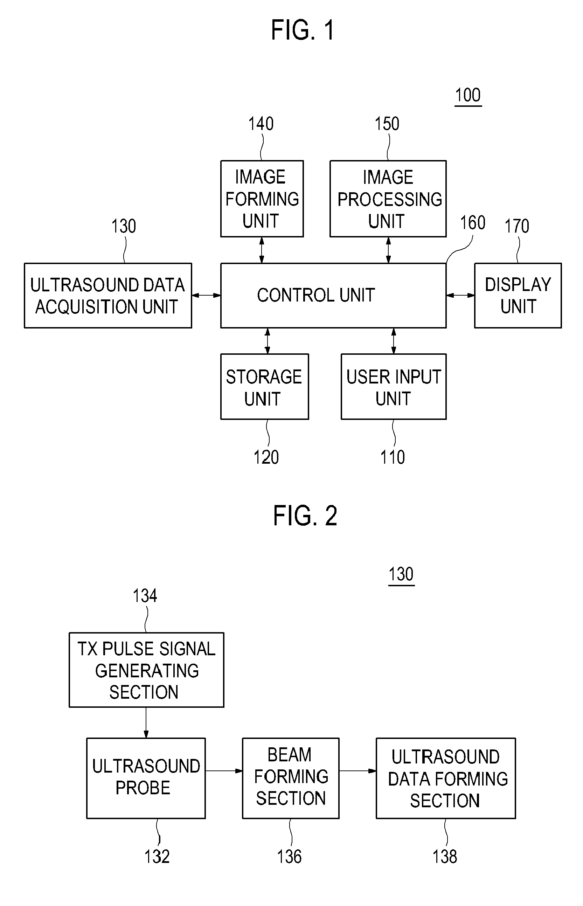

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure. Referring to FIG. 1, an ultrasound system 100 constructed in accordance with one embodiment is shown. The ultrasound system 100 may include a user input unit 110 configured to receive user instructions. The user instructions may include a first user instruction to define a region of interest (ROI) and a second user instruction to select a seed point. The user input unit 110 may be a mouse, a keyboard, a track ball, a touch screen and the like. The ultrasound system 100 may further include a storage unit 120 for storing templates for use to detect an image of a target object (e.g., vessel) in an ultrasound image.

The ultrasound system 100 may further include an ultrasound data acquisition unit 130. The ultrasound data acquisition unit 130 may be configured to transmit ultrasound beams to a target object and receive ultrasound echoes reflected from the target object to thereby form ultrasound data representative of the target object.

The ultrasound data acquisition unit 110 may include an ultrasound probe 132 having an array transducer 132A. The array transducer 132A contains a plurality of transducer elements that may be operable to transmit ultrasound beams along scan lines, which may be aligned from a scan head of the ultrasound probe 132 to the target object. The scan lines may be steered at one of multiple angles relative to the scan head of the ultrasound probe 132. The ultrasound probe 132 may include any one of a linear probe, a convex probe and the like.

In one embodiment, the transmission of the ultrasound beams may be controlled by a transmission (Tx) pulse generating section 134, which is coupled to the ultrasound probe 132. The Tx pulse generating section 134 may include a plurality of pulsers to generate Tx pulses, which are delivered to the transducer elements of the array transducer 132A for actuation thereof. The Tx pulse generating section 134 may be further operable to apply delays to the Tx pulses to form a Tx pattern, with which to control the actuation of the transducer elements. In one embodiment, the delays applied to the Tx pulses may be determined by considering virtual transducer elements, which may be virtually formed and extended from one of edges of the array transducer 132A in a longitudinal direction of the array transducer 132A. In this way, the ultrasound beam may be transmitted at a predetermined steering angle. The transducer elements of the ultrasound probe 132 may receive ultrasound echoes reflected from the target object and then output electrical receive signals, which may be analog signals.

The ultrasound data acquisition unit 110 may further include a beam forming section 136, which is coupled to the ultrasound probe 132. The beam forming section 136 may be operable to digitize the electrical receive signals to obtain digital signals. The beam forming section 136 may be further operable to apply delays to the digital signals in consideration of distances between the elements of the ultrasound probe 132 and focal points and the steering angles of the scan lines. The beam forming section 136 may be further operable to sum the delayed digital signals to form receive-focused beams. The beam forming section 136 may be also operable to perform compensation processing upon the receive-focused beams by considering virtual transducer elements. In one embodiment, the compensation processing may include scan line gain compensation and time gain compensation.

The ultrasound data acquisition unit 110 may further include an ultrasound data forming section 138, which is coupled to the beam forming section 136. The ultrasound data forming section 138 may be operable to form ultrasound data based on the receive-focused beams. The ultrasound data may include radio frequency data, Inphase/Quadrature data and the like.

The ultrasound system 100 may further include an ultrasound image forming unit 140, which is coupled to the ultrasound data forming section 138. The ultrasound image forming unit 140 may be operable to form ultrasound images based on the ultrasound data. In one embodiment, the ultrasound images may include a brightness-mode (B-mode) image, although they are not limited thereto.

The ultrasound system 100 may further include an image processing unit 150, which is coupled to the ultrasound image forming unit 140. The image processing unit 150 may be operable to detect a center of the target object (e.g., vessel), which constitutes the main part of the ultrasound image. The center detection may be achieved by using the vessel template stored in the storage unit 120 or based on the user instruction.

The ultrasound system 100 may further include a control unit 150, which is coupled to elements of the ultrasound system 100 such as the user input unit 110, the storage unit 120, the ultrasound acquisition unit 130, the image forming unit 140 and the image processing unit 150. The control unit 140 may be responsive to the user instruction to issue first control signals to control the operations of elements of the ultrasound system 100. The control unit 140 may be further operable to issue second control signals to form the virtual transducer elements, detect the vessel center and define a steering angle of scan lines.

In the embodiment explained above, it has been described that the ultrasound image forming unit 140, the image processing unit 150 and the control unit 160 are configured with separate elements in the ultrasound system 100. However, these components may be embodied with a single processor such as a central processing unit, a microprocessor, a graphic processing unit, application-specific integrated circuit and the like.

The ultrasound system may further include a display unit 170 for displaying the ultrasound images such as the elastic images, the B-mode images, the compound images and the like. In one embodiment, the display unit 160 may include at least one of a cathode ray tube (CRT) display, a liquid crystal display (LCD), an organic light emitting diode (OLED) display and the like.

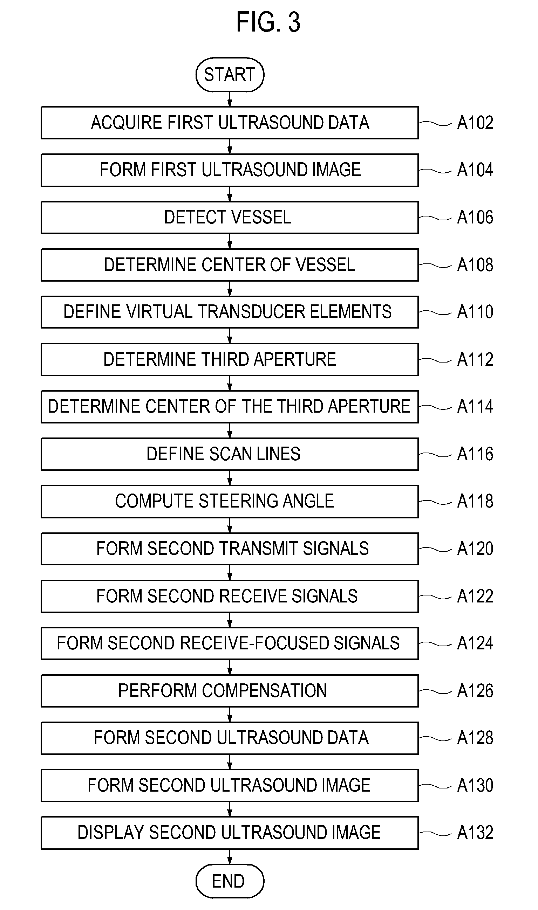

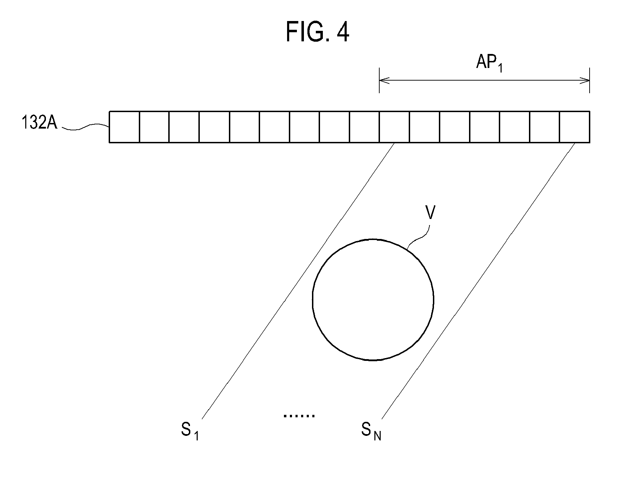

Hereinafter, a process of forming an ultrasound image by adjusting a steering angle of scan lines based on the virtual transducer elements will be explained by referring to the figures. Referring to FIG. 3, the ultrasound data acquisition unit 130 may be operable to transmit ultrasound signals to a target object and receive echo signals, thereby acquiring first ultrasound data at act A102. More particularly, the Tx signal forming section 134 may be operable to apply Tx signals by considering transducer elements included in a predetermined aperture ("first aperture AP₁") and predetermined focal points to thereby output first Tx signals, as shown in FIG. 4. The aperture may represent a range of transducer elements, which may substantially participate in transmission and reception of the ultrasound signals. In FIG. 4, a symbol "V" may represent a target object image, i.e., a vessel image in the ultrasound image and symbols S₁-S_{N} may represent scan lines originating from the first aperture AP₁. The ultrasound probe 132 may be operable to transmit ultrasound signals to the target object in response to the first Tx signals. It may then receive echo signals to thereby form first receive signals. The beam forming section 136 may be operable to focus the first receive signals in consideration of the transducer elements within the first aperture AP1 and the predetermined focal points to form first receive-focused beams. The ultrasound data forming unit 138 may be operable to form first ultrasound data by using the first receive-focused beams.

The image forming unit 140 may be operable to form a first ultrasound image based on the first receive-focused beams at act A104. The first ultrasound image may be displayed on a screen of the display unit 170. The image processing unit 150 may be operable to detect the vessel in the first ultrasound image at step A106 and then detect a center of the vessel at step A108.

In one embodiment, the image processing unit 150 may be operable to extract a vessel template from the storage unit 120. The image processing unit 150 may be operable to position the extracted vessel template on the first ultrasound image and move the vessel template to detect the vessel in the first ultrasound image. The vessel detection may be carried out by using a well-known method such as pattern patting, sum of absolute difference and the like. The image processing unit 130 may be operable to detect a maximum diameter from the detected vessel and define a center of the maximum diameter as a center of the vessel.

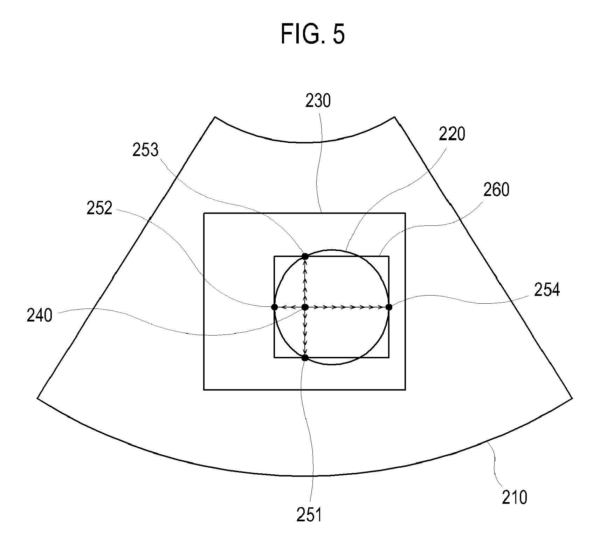

In one embodiment, if a first user instruction for defining a region of interest is inputted through the user input unit 110, then the image processing unit 150 may be operable to indicate a region of interest 230 on an ultrasound image 210. The image processing unit 150 may be operable to determine a center of the region of interest 230 and indicate the determined center of the region of interest 230 as a point 240 on the ultrasound image 210, as shown in FIG. 5. The image processing unit 140 may be operable to move the center point 240 in up, down, right and left directions by predetermined distances to detect regions 251-254 having maximum brightness differences at the respective directions. The image processing unit 140 may determine the detected regions 251-254 as a vessel wall 220. The image processing unit 140 may be operable to form a rectangle whose sides pass through the regions 251-254. The image processing unit 140 may determine the center of the rectangle as a center of the vessel 220.

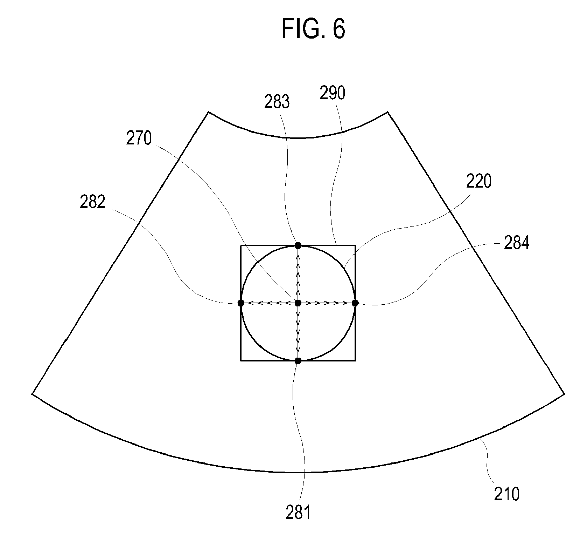

In one embodiment, if a second user instruction for defining a seed point is inputted through the user input unit 110, then the image processing unit 150 may be operable to indicate a seed point 270 on the first ultrasound image 210, as shown in FIG. 6. The image processing unit 150 may be operable to move the seed point 270 in up, down, right and left directions by predetermined distances to detect regions 281-284 having maximum brightness differences at each of the directions. The detected regions 281-284 may be determined as walls of the vessel 220. The image processing unit 140 may be operable to form a rectangle, of which each side passes through the regions 281-284. The image processing unit 140 may determine a center of the rectangle as the center of the vessel 220.

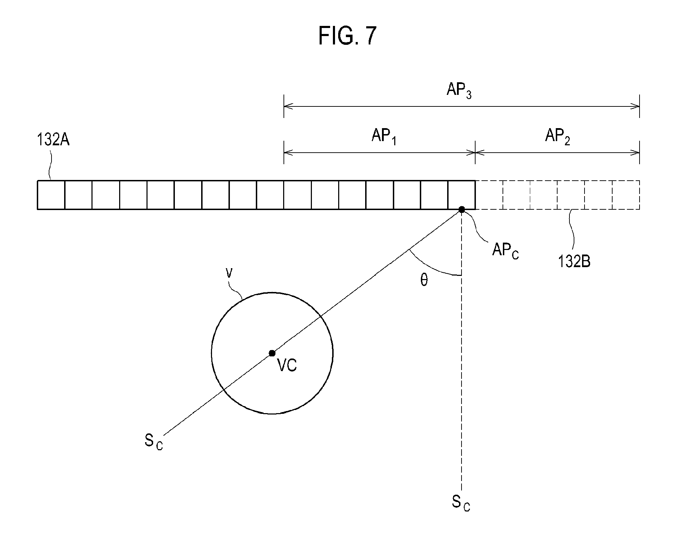

The control unit 160 may be operable to define a plurality of virtual transducer elements 132B (denoted by dotted lines) from one of the edges of the array transducer 132A in a longitudinal direction thereof, as show in FIG. 7. The virtual transducer elements may be defined manually by inputting a user instruction or automatically according to preset information stored in the ultrasound system 100. The control unit 160 may be further operable to determine a second aperture AP₂ corresponding to the virtual transducer elements 132B and a third aperture AP₃ including the first aperture AP₁ and the second aperture AP₂.

The control unit 160 may be operable to compute a center AP_{C} of the third aperture AP₃ at act A114 and define a scan line S_{C} originating from the center AP_{C} at step A116. The control unit 160 may be operable to further compute a steering angle θ of the scan line S_{C}, which passes the vessel center VC, at step A118. This computed steering angel θ may be set as a maximum steering angle.

The Tx pulse generating section 134 may be operable to generate Tx pulses. The Tx pulse generating section 134 may be further operable to apply delays to the Tx pulses in consideration of the positions of transducer elements within the third aperture AP₃, a focal point (i.e., vessel center) and the steering angle θ computed at act A118 to thereby output second Tx signals at actA120.

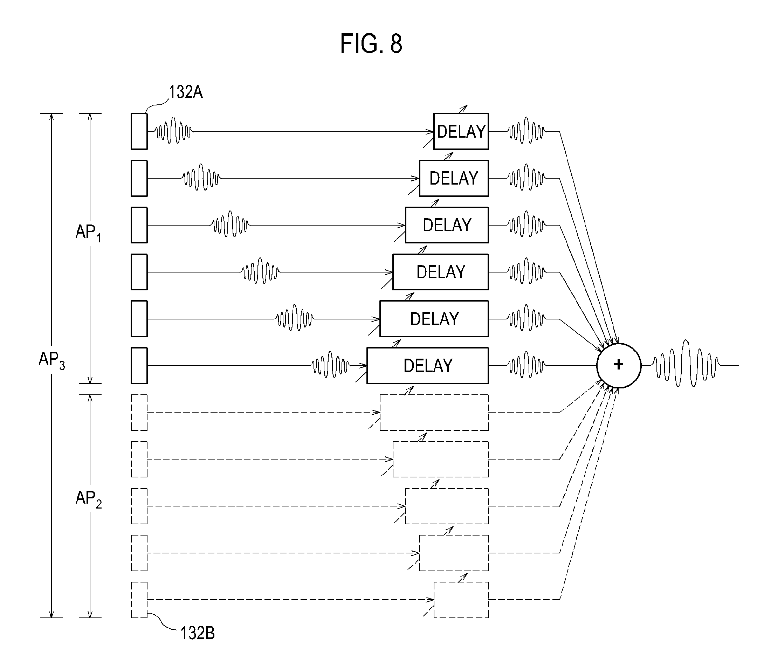

The ultrasound probe 132 may be operable to output ultrasound signals to the target object in response to the second Tx signals and receive echo signals reflected from the target object to thereby form second receive signals at act A122. The beam forming section 136 may be operable to apply delays to the second receive signals by considering the positions of transducer elements within the third aperture AP₃, a focal point and the steering angle θ to thereby form second receive-focused beams at step A124. In one embodiment, the receive signals may include signals, which are outputted from the real transducer elements 132A, and signals that are virtually outputted from the virtual transducer elements 132B, as shown in FIG. 8. The beam forming section 136 may be further operable to perform scan line gain compensation upon the second receive-focused beams based on the virtual transducer elements 132B. In this way, a relatively low intensity of the second receive-focused beams due to the virtual transducer elements may be compensated. Further, the beam forming section 136 may be further operable to perform time gain compensation upon the second receive-focused beams for compensating for attenuation of the ultrasound signals. The ultrasound data forming section 138 may be operable to form second ultrasound data based on the second receive-focused beams provided from the beam forming section 136 at step A128. The image forming unit 150 may be operable to form a second ultrasound image by using the second ultrasound data provided from the ultrasound data forming section 138 at step A130. The display unit 170 may display the second ultrasound image, which may be provided from the image forming unit 150, at step A132.

Although embodiments have been described with reference to a number of illustrative embodiments thereof, it should be understood that numerous other modifications and embodiments can be devised by those skilled in the art that will fall within the scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
an ultrasound data acquisition unit configured to transmit ultrasound signals to a target object along first scan lines steered at a first steering angle and receive echo signals reflected from the target object to form first ultrasound data, the ultrasound data acquisition unit including an array transducer containing a plurality of transducer elements;
a processing unit configured to form a first ultrasound image by using the first ultrasound data and determine a center of the target object on the first ultrasound image; and
a control unit configured to define virtual transducer elements associated with the array transducer, define second scan lines and compute a second steering angle of the second scan lines based on the virtual transducer elements and the center of the target object,
wherein the ultrasound data acquisition unit is further configured to transmit ultrasound signals to the target object along the second scan lines steered at the second steering angle and receive echo signals reflected from the target object to form second ultrasound data, and
wherein the processing unit is further configured to form a second ultrasound image by using the second ultrasound data.

2. The ultrasound system of Claim 1, wherein the control unit is configured to define the virtual transducer elements extended from one of edges of the array transducer in a longitudinal direction of the array transducer.

3. The ultrasound system of Claim 2, wherein the control unit is configured to:
determine a first aperture including predetermined transducer elements participating in forming the first ultrasound data;
define the first scan lines originating from the first aperture;
determine a second aperture including the virtual transducer elements;
determine a third aperture including the first and second apertures;
compute a center of the third aperture; and
compute the second steering angle of the second scan lines passing the center of the target object and originating from the third aperture, wherein the second steering angle is larger than the first steering angle.

4. The ultrasound system of Claim 3, wherein the ultrasound data acquisition unit includes:
a transmit pulse signal generating section configured to generate transmit pulse signals and apply delays to the transmit pulse signals;
an ultrasound probe including the array transducer containing the transducer elements and being configured to generate the ultrasound signals in response to the transmit pulse signals and receive the echo signals to output the receive signals;
a beam forming section operable to apply delays to the receive signals to form receive-focused beams and perform compensation processing upon the receive-focused beams by considering the virtual transducer elements and the steering angles; and
an ultrasound data forming section configured to form the first and second ultrasound data by using the receive-focused beams.

5. The ultrasound system of Claim 1, further comprising a storage unit for storing a template of the target object for detection, wherein the processing unit is configured to:
extract the template from the storage unit;
position the extracted template on the first ultrasound image;
move the template to detect the target object from the first ultrasound image;
detect a maximum diameter from the detected target object; and
determine a center of the maximum diameter as the center of the target object.

6. The ultrasound system of Claim 1, further comprising a user input unit for allowing a user to input a user instruction for defining a region of interest on the first ultrasound image, wherein the processing unit is configured to:
define a region of interest on the first ultrasound image in response to the user instruction;
determine a center point of the region of interest on the first ultrasound image;
move the center point in up, down, right and left directions, respectively, by predetermined distances to detect regions, each having a maximum brightness at respective directions;
determine the detected regions as walls of the target object;
form a virtual rectangle, of which each side passes through the regions,
respectively; and
determine a center of the virtual rectangle as the center of the target object.

7. The ultrasound system of Claim 1, further comprising a user input unit for allowing a user to input a user instruction for defining a seed point on the first ultrasound image, wherein the processing unit is configured to:
indicate a seed point on the first ultrasound image;
move the seed point in up, down, right and left directions by predetermined distances to detect regions having maximum brightness differences at the respective directions;
determine the detected regions as walls of the target object;
form a virtual rectangle, whose sides pass through the regions, respectively; and
determine a center of the virtual rectangle as the center of the target object.

8. A method of forming an ultrasound image by adjusting a steering angle of scan lines using virtual transducer elements in an ultrasound system having an array transducer containing a plurality of transducer elements, the method comprising:
a) transmitting ultrasound signals to a target object along first scan lines originating from a first aperture including predetermined transducer elements and steered at a first steering angle, and receiving echo signals reflected from the target object to form first ultrasound data;
b) forming a first ultrasound image by using the first ultrasound data and determine a center of the target object on the first ultrasound image;
c) defining virtual transducer elements associated with the array transducer, defining second scan lines and computing a second steering angle of the second scan lines based on the virtual transducer elements and the center of the target object;
d) transmitting ultrasound signals along the second scan lines originating from a second aperture including the predetermined transducer elements and the virtual transducer elements and steered at the second steering angle into the target object, and receiving echo signals reflected from the target object to form second ultrasound data; and
e) forming a second ultrasound image by using the second ultrasound data.

9. The method of Claim 8, wherein the virtual transducer elements are defined to be extended from one of edges of the array transducer in a longitudinal direction of the array transducer.

10. The method of Claim 9, wherein the step c) includes:
computing a center of the second aperture; and
computing the second steering angle of the second scan lines passing the center of the target object, wherein the second steering angle is larger than the first steering angle.

11. The method of Claim 10, wherein the step d) further comprises performing compensation processing upon the receive-focused beams by considering the virtual transducer elements and the steering angles.

12. The method of Claim 8, further comprising storing a template of the target object for detection in a storage unit, wherein the step b) includes:
extracting the template from the storage unit;
positioning the extracted template on the first ultrasound image;
moving the template to detect the target object from the first ultrasound image;
detecting a maximum diameter from the detected target object; and
determining a center of the maximum diameter as the center of the target object.

13. The method of Claim 8, further comprising inputting a user instruction for defining a region of interest on the first ultrasound image, wherein the step b) includes:
defining a region of interest on the first ultrasound image in response to the user instruction;
determining a center point of the region of interest on the first ultrasound image;
moving the center point in up, down, right and left directions by predetermined distances to detect regions having maximum brightness differences at the respective directions;
determining the detected regions as walls of the target object;
forming a virtual rectangle, of which each side passes through the regions, respectively; and
determining a center of the virtual rectangle as the center of the target object.

14. The method of Claim 8, further comprising inputting a user instruction for defining a seed point on the first ultrasound image, wherein the step b) includes:
indicating a seed point on the first ultrasound image;
moving the seed point in up, down, right and left directions by predetermined distances to detect regions having maximum brightness differences at the respective directions;
determining the detected regions as a wall of the target object;
forming a virtual rectangle, of which each side passes through the regions, respectively; and
determining a center of the virtual rectangle as the center of the target object.
